# EUROPEAN PATENT SPECIFICATION

(11) **EP 2 872 204 B1**
(45) Date of publication and mention of the grant of the patent: **09.09.2020**
(21) Application number: 13734457.8
(22) Date of filing: 09.07.2013
(51) Int. Cl.: A61M 5/46, A61M 5/32, A61M 5/20

(54) **AUTOINJECTOR**
AUTOINJEKTOR
AUTOINJECTEUR

(30) Priority: 11.07.2012 EP 12176029
(43) Date of publication of application: 20.05.2015
(73) Proprietor: Novartis AG, 4056 Basel (CH)
(72) Inventor: BRYANT, Andrew, 4002 Basel (CH); BUETTGEN, Heinrich, 4002 Basel (CH)
(74) Representative: Stuttle, James Henry Phillip
(86) International application number: PCT/EP2013/064510
(87) International publication number: WO 2014/009382

(56) References cited:
- WO-A2-99/30759
- WO-A2-2006/062788
- US-A1- 2002 072 709
- US-A1- 2003 236 502
- US-A1- 2008 154 198
- US-A1- 2009 259 180

## Description

The present invention relates to an injection device, specifically an injection device comprising a needle having a removable needle shield, a container, such as a syringe, and an injector body.

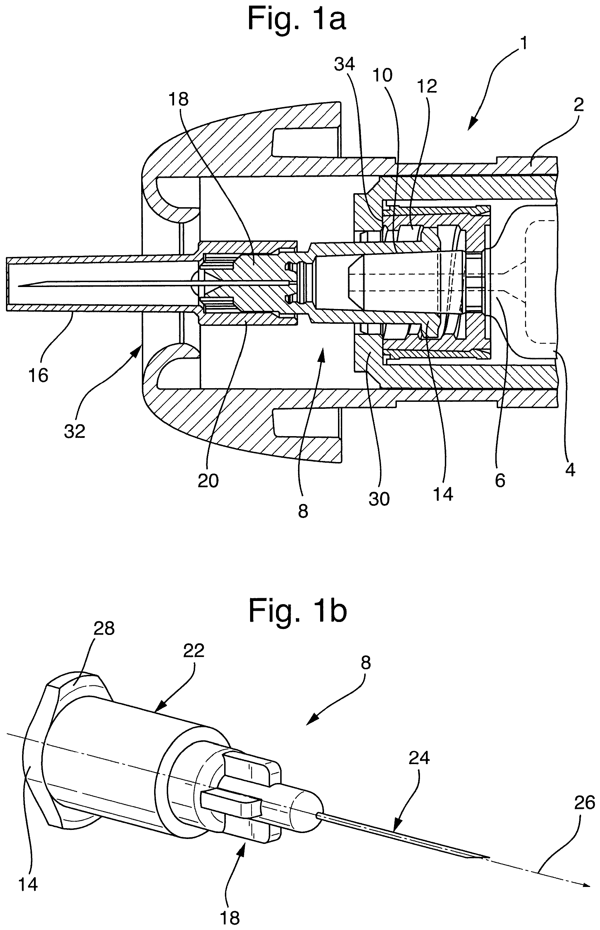

Controlling depth of injection is important to ensure that the drug is delivered to the correct tissue. During a manual injection the user has direct control of the injection depth. However, when using an injection device, such as a autoinjector, the user may be able to select a desired depth of injection, but it is the device that directly controls the injection depth. The depth of injection is often controlled by a stop within the injection device that interacts with part of the drug container, for example a syringe, to limit injection depth. However, there are various components within the tolerance chain of prior art autoinjectors that can result in a potentially significant variability in injection depth. A prior art syringe is shown in Figures 1a and 1b.

Figure 1a shows an end of an injector 1, in this case an autoinjector which includes a body 2 and a syringe 4. The syringe 4 includes an outlet 6 to which an injection needle 8 (better shown in Figure 1b) is coupled. In this case the outlet 6 comprises a luer type connector 10 with a screw thread 12 into which a connection end 14 of the injection needle 8 is screwed. The injection needle 8 is protected by a needle shield 16 which is coupled to a shield portion 18 of the hub 22 of injection needle 8 by a connection sleeve 20.

As can be seen in Figure 1b, the injection needle 8 comprises a hub 22 and a needle 24 which extend along a first axis 26. A flange 28 is provided at the connection end 14 for engaging with the screw thread 12.

The autoinjector 1 also includes a body stop 30. The body stop 30 is arranged a predetermined distance from an injection opening 32. The body stop 30 comprises an annular shoulder 34 around an opening through which the needle 24, the needle shield 16 and the hub 20 are able to pass, but through which the screw part 12 of the luer type lock 10 cannot pass. This means that the distance between the syringe 4 and injection opening 28 is controlled by the body stop 30.

WO 2006/062788 A2 discloses an injection needle according to the preamble of claim 1.

The present invention provides an injector comprising a body and a container, the body having an injection opening and including a drive mechanism which can be actuated to move the container within the body and inject medication from the container through the injection opening, the container comprising an outlet and including an injection needle releasably coupled thereto, the injection needle comprising a hub and a needle, the hub extending along a first axis between a connecting end and a needle end, the connection end comprising connection means for connecting to the container and the needle connecting to the hub at a needle end, the needle extending from the hub along the first axis to an injection end, the injection needle comprising a channel extending from the connection end of the hub to the injection end of the needle, the hub comprising a reference shoulder extending radially from the hub and directed towards the injection end, the injection needle further comprising a needle shield, the needle shield releasably coupled to the needle hub and substantially covering the needle, the body further including a body stop, the body stop comprising a shoulder extending radially inwardly from the body to define an aperture though which the needle shield, but not the reference shoulder can pass, the body stop being configured to contact the at least one reference shoulder extending from the hub to limit the distance the injection end of the needle extends out of the injection opening.

By providing a forward directed, (i.e. away from the connection end and towards the injection end), reference shoulder on the hub a tighter control over the distance that an injection end of the needle extends through an injection opening of an injector is made possible. The reference shoulder on the hub results in fewer components in the axial tolerance chain since, for example, the axial tolerances of the depth of connection of the luer engagement between the injection needle and container is removed. Such tolerances can be quite large, especially in cases where diameter tolerances in the luer cone dimensions are amplified by a small cone angle. It is also possible that the change in load path may reduce the chance of injector needle flange breakage in an autoinjector in which the forces can be quite high. This sort of tolerance chain consideration is particularly relevant for removable needles since needles permanently attached to the container do not suffer from the attachment tolerances mentioned above.

The injector body provides a housing adapted to contain the container and cover some or all of the container. The injection opening is an opening into the body through which the needle attached to the container within the injector body can be caused to protrude so that it can penetrate the body of a human or animal to deliver medication thereto.

It should be noted that the term "container" herein includes syringes, cartridges and other medicament delivery or containing devices to which an injection needle can be, or is, attached and which include a variable volume chamber sealed by a movable stopper. In a preferred embodiment the container is a syringe which is filled with a medicament and then inserted into the injector. Having the body stop configured as an aperture through which the shield can pass, but not the reference stop means that the shielded needle can be attached to the container and the container and needle assembly subsequently inserted into the injector body such that the needle shield extends from the injection outlet. This means that the assembly is 'safe' as the needle is shielded throughout. The needle shield can then be removed by a user through the injection outlet prior to use without risking contact with the needle. The container may be filled, either pre-filled or filled by a user prior to assembling the container into the injector body, with a medicament intended for injection into the body of a human or animal patient. In one embodiment the medicament is interferon beta-1b.

It should be noted that there are many suitable drive mechanisms that should be used in the injector of the invention. In one embodiment the drive mechanism comprises one or more mechanical springs that can be primed and subsequently released to drive the container and attached needle towards the patient so that the needle pierces the skin and the drive mechanism then causes he medicament within the container to be forced through the needle and thereby delivered to the patient. In other embodiments one or more of the functions of causing the needle to pierce the skin, causing the injection of medicament through the needle or causing other movements of parts of the injector can be accomplished or assisted using motors, gas springs or other mechanisms.

In one embodiment the injection needle may be substantially standard with the exception of the addition of the reference shoulder. The hub may be fabricated for any suitable material, for example a plastic, metal, glass or combination thereof. The needle may be any suitable material, for example a surgical grade metal.

The connection end of the hub may include a radial flange for connection to an internal screw fitting on a container to facilitate connection of the injection needle to the container. The connection end of the hub may include an opening into a female part of a luer type connector.

The needle may be connected to the hub in any suitable way (many are known in the art), for example glued or staked. The injection end of the needle may be sharpened to facilitate piercing the skin or other target material. Such sharpening can take many forms and is well known in the art.

The injection needle comprises a channel extending from the connection end of the hub to the injection end of the needle to allow fluid from within a container connected to the injection needle to be expelled through the injection end of the needle. The channel may be located substantially centrally through the injection needle and may extend substantially along the first axis, although deviations from the first axis or non-central channels are also possible.

The reference shoulder extending radially from the hub and directed towards the injection end may be formed from any suitable material. The reference shoulder is formed by attaching a component to the hub. The reference shoulder need not be fabricated from the same material as the hub. The reference shoulder must be securely coupled to the hub so that it substantially cannot move in the axial direction as this ensures that the location of the shoulder remains substantially as intended and the injection depth is therefore consistent and substantially as intended.

In one embodiment the hub extends for a length L along a first axis between the connecting end and the needle end and the at least one reference shoulder may be located less than a distance of 3L/4 from the needle end, or less than 5L/8 from the needle end, or less than L/2 from the needle end. In the same, or other embodiments, the at least one reference shoulder may be located at least a distance of L/6 from the needle end, or at least a distance of L/4 from the needle end.

The needle hub may include a shield stop to prevent the needle shield from being pushed too far onto the hub. The shield stop is a shoulder extending perpendicular to the first axis. The shield stop may be located between the reference stop, and the needle end of the hub. In another embodiment the shield stop and the reference stop may be located at substantially the same axial location on the hub.

The reference shoulder is provided on a sleeve extending around the hub. The sleeve is a separate component added to an injection needle, for example a substantially standard injection needle. The sleeve is substantially unable to move away from the injection end such that the reference shoulder is in a fixed axial position relative to the injection end. The sleeve may be a friction fit to the hub or may be held in place using a positive connection such as a snap-fit, a screw-fit. The sleeve could be held in place with an adhesive. The sleeve could also be fabricated integrally with the hub, for example by making the hub wall thicker and providing the reference shoulder in that way.

In one embodiment the distance between the injection end and the reference shoulder may be less than 30mm, may be less than 25mm, less than 20mm or less than 15mm.

The injection needle is provided with a removable needle shield releasably coupled to a shield portion adjacent the connection end. The needle shield may be coupled to the shield portion by a sleeve or any other suitable means. The reference shoulder may extend radially beyond the radial extent of the sleeve of the needle shield and/or the radial extent of a shield stop on the hub.

In one embodiment the reference shoulder may extend radially at least 0.2mm, or at least 0.3mm, or at least 0.5mm or at least 1mm away from the first axis. If a shield stop is provided, the reference shoulder may extend radially at least 0.2mm, or at least 0.3mm, or at least 0.5mm or at least 1mm radially beyond the shield stop. The reference stop may extend to an outer diameter of at least 5mm, at least 6mm, at least 6.5mm.

In one embodiment of the injector the distance between the body stop and the injection opening may adjustable. Such adjustment may be provided in many ways, for example those known in the art. The adjustment may be by sliding adjustment, screw adjustment, rack and pinion adjustment or other suitable mechanisms.

It should be understood that throughout this specification and in the claims that follow, unless the context requires otherwise, the word "comprise", or variations such as "comprises" or "comprising", implies the inclusion of the stated integer or step, or group of integers or steps.

The invention will now be further described, by way of example only, with reference to the following drawings in which:
Figures 1a and 1b show prior art injectors and injection needles;
Figure 2a and 2b show an injector before and after an injection event;
Figure 3 shows an injection needle including a reference shoulder provided on an annular flange;
Figure 4 shows an injection needle including a reference shoulder provided on axial ribs;
Figure 5 shows an injection needle including a reference shoulder provided on a sleeve;
Figure 6 shows an injection needle including a reference shoulder provided on a different sleeve.

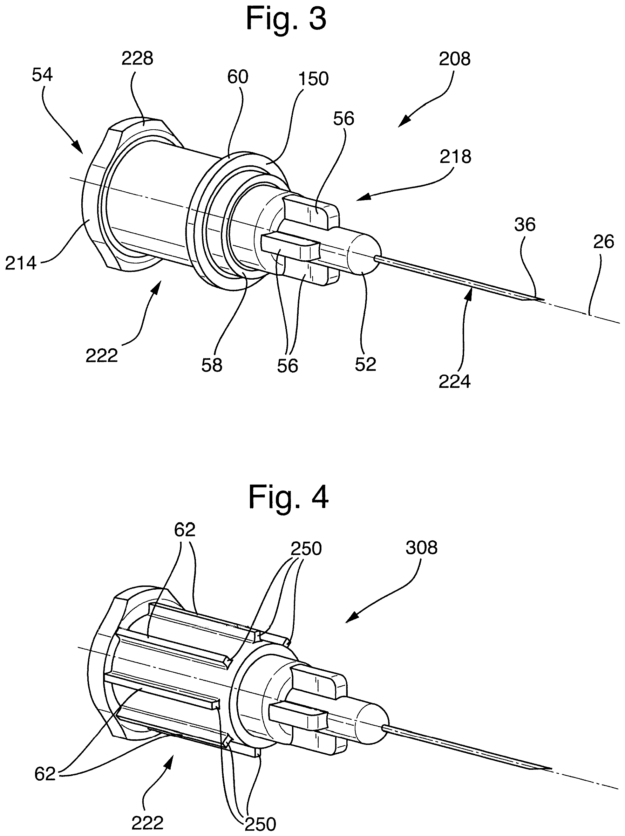

Figures 3 and 4 show embodiments that do not fall within the scope of the claims.

Figures 1a and 1b show prior art injection needles and assemblies and have already been discussed.

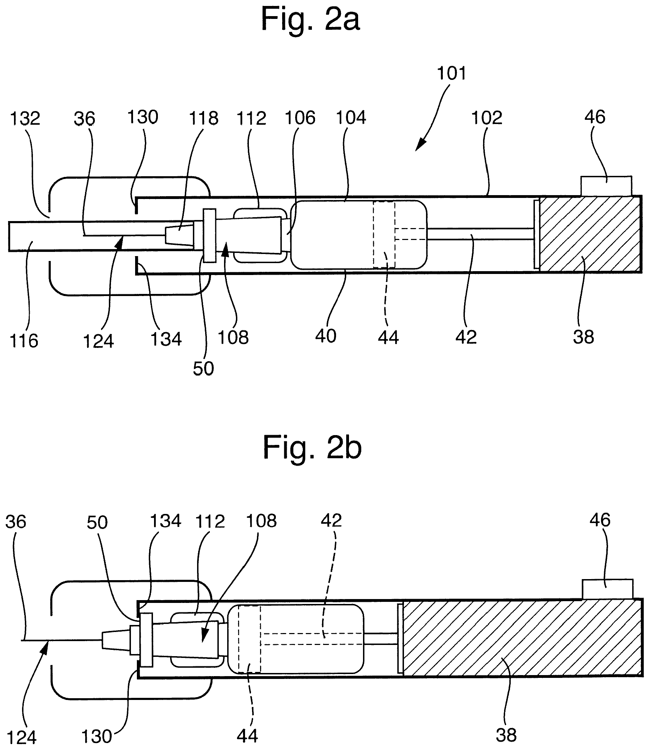

Figure 2a shows an injector 101 in a primed condition which includes a body 102 and a container, in this case a syringe 104. An injection needle 108 is coupled to an outlet 106 of the syringe 104. At a forward end of the injector 101 there is provided an injection opening 132.

In the primed condition the syringe 101 is arranged within the body such that an injection end 36 of the needle 124 is arranged within the body 102. A drive mechanism 38, for example a spring or other suitable mechanism is arranged within the body 102 at an end opposite the injection opening 132. A button 46 is arranged to actuate the drive mechanism 38.

The syringe 104 comprises a syringe body 40, a plunger 42 and a stopper 44. The syringe body 40 includes an outlet 106 to which the injection needle 108 is coupled.

In the primed state a needle shield 116 is attached to a shield portion 118 of the needle hub 108.

Figure 2b shows the syringe 101 in a post-delivery condition. The drive mechanism 38 has extended towards the injection opening 132 causing the syringe 104 to move towards the injection opening 132 until such movement is prevented. The movement of the syringe 104 towards the injection opening 132 is prevented when a reference shoulder 50 on the hub 108 makes contact with the body stop 130. The body stop is an annular shoulder 134 within the body 102 arranged a predetermined distance away from the injection opening 132. The predetermined distance being such that when the reference shoulder 50 is in contact with the body stop 130 the injection end 36 of the needle 124 extends from the body 102 through the injection opening 132. The drive mechanism 38 has also caused the stopper 44 to move towards the outlet 106 causing the fluid previously within the syringe 104 to be expelled through the injection needle 108. The drive mechanism 38 first causes the syringe 104 to move to the body stop and then causes the stopper to move towards the outlet 106. This sequence of actions causes the injection end 36 of the needle 124 to pierce the skin and then deliver the fluid from within the syringe 104. The fluid may be an injectable medicament.

Figure 3 shows an injection needle 208 comprising a hub 222 and a needle 224. The hub 222 extending along a first axis 26 between a connecting end 214 and a needle end 52. The connection end 214 comprises connection means for connecting to a syringe. In this case the connection means comprises a flange 228 and an opening into a female luer type connection 54. The needle 224 connecting to the hub 222 at a needle end 52 and extending from the hub 222 along the first axis 26 to an injection end 36 which is sharpened. The injection needle 208 comprising a channel extending from the connection end 214 of the hub 222 to the injection end 52 of the needle 224. The hub 222 also comprises a reference shoulder 150 extending radially from the hub 222 and directed towards the injection end 36.
The shield portion 218 of the hub 222 comprises four axial shield ridges 56 that engage with the sleeve 20 of a needle shield 16. This means that a user can hold the injection needle 208 by the needle shield, connect it to a syringe 4 and screw it to a screw thread such as that shown in Figure 1a as 12 or in Figures 2a and 2b as 112. The needle shield 16 is prevented from being pushed too far onto the hub 222 by a shield stop 58. The shield stop 58 is a shoulder extending perpendicular to the first axis 26.
In this example the reference shoulder 150 is provided by a circumferential ridge or flange 60 extending around the hub 222. The reference shoulder 150 extends radially beyond the extent of the shield stop 58.

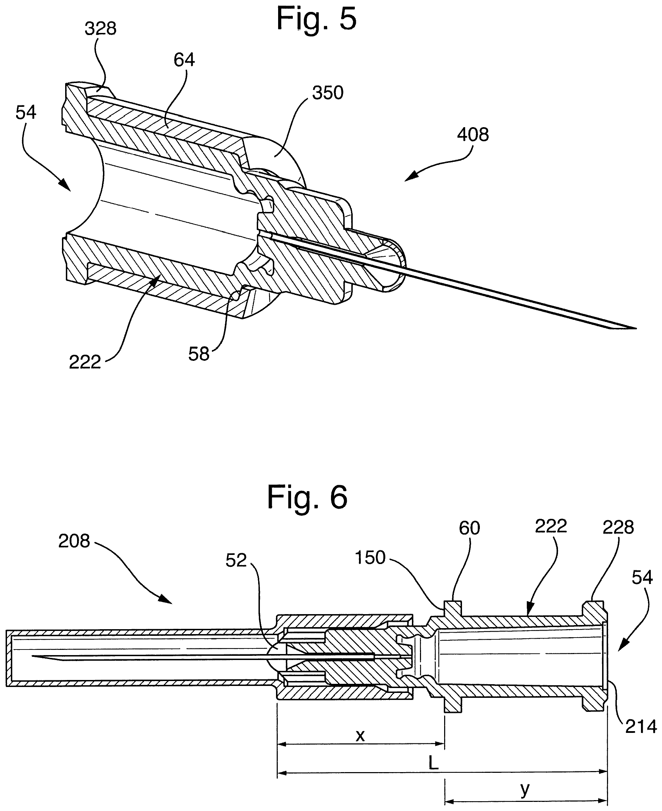

Figure 4 shows a different injection needle 308. The injection needle 308 is substantially the same as that shown in Figure 3 except that reference shoulders 250 are provided on a plurality of axially extending hub ridges 62. There are between nine and eleven axially extending hub ridges 62 which are substantially evenly circumferentially distributed around the hub 222. Figure 5 shows a further injection needle 408. The injection needle 408 is substantially the same as that shown in Figure 3 except that, in accordance with the present invention, reference shoulder 350 is provided on a sleeve 64 that is fitted to, and substantially surrounds, the hub 222. The sleeve 64 covers the shield stop 58, thereby creating a new shield stop 158 closer to the injection end by the thickness of the sleeve in the shield stop area. In this embodiment the new shield stop 158 and reference shoulder 350 are in substantially the same axial location. The sleeve 64 extends to the flange 328. The sleeve 64 is a separate component and is fitted to the hub 222 after manufacture of the hub 222.

Figure 6 shows an injection needle 508. The injection needle 508 includes a sleeve 164 which provides the reference shoulder 450. The injection needle 508 and sleeve 164 are substantially the same as that shown in Figure 5. The sleeve 164 is shorter than the sleeve 64 of Figure 5, in that it covers the shield stop 58 and extends along the hub 222 towards, but not reaching the flange 328. At an end 70 of the sleeve 164 there is an outwardly directed flange 72 which may assist with automated handling of the sleeve 164 during production. Such a flange 72 could be added to any sleeve.

In Figures 3, 4, 5, and 6 the injection needles have substantially the same key dimensions. From the connection end 214 to the reference stop 150, 250, 350 is between 7.5 to 8.5mm. From the connection end 214 to the needle end 52 is between 16 to 17mm.

Figure 6 shows a cross section of the injection needle 208 of Figure 3. The hub 222 extends for a length L along a first axis 26 between the connecting end 214 and the needle end 52. The reference shoulder 150 is located a distance x from the needle end and a distance y from the connection end. In this case the distance x is about L/2 and the distance y is about L/2.

It should be understood that the invention has been described above by way of example only and that modifications in detail can be made without departing from the scope of the claims.

## Claims

1. An injector comprising a body (102) and a container (104), the body (102) having an injection opening (32) and including a drive mechanism (38) which can be actuated to move the container (104) within the body (102) and inject medication from the container (104) through the injection opening (32), the container (104) comprising an outlet (106) and including an injection needle (108) releasably coupled thereto, the injection needle (108) comprising a hub (222) and a needle (224), the hub (222) extending along a first axis between a connecting end (214) and a needle end (52), the connection end (214) comprising connection means for connecting to the container (104) and the needle (224) connecting to the hub (222) at the needle end (52), the needle (224) extending from the hub (222) along the first axis to an injection end (52), the injection needle (108) comprising a channel extending from the connection end (214) of the hub (222) to the injection end (52) of the needle (224), the needle (224) further comprising a needle shield (16), the needle shield (16) releasably coupled to the needle hub (222) and substantially covering the needle (224), the body (102) further including a body stop (130), the body stop (130) comprising a shoulder (134) extending radially inwardly from the body (130), wherein the shoulder (134) of the body stop (130) defines an aperture though which the needle shield (16), but not a reference shoulder (350) can pass, the body stop (130) being configured to contact the reference shoulder (350) to limit the distance of the injection end (52) of the needle (224) extending out of the injection opening (32), **characterised in that** the reference shoulder (350) is provided on a sleeve (64) that is fitted to and substantially surrounds the hub (222), the sleeve (64) being a separate component added to the injection needle and substantially unable to move away from the injection end (52) such that the reference shoulder (350) is in a fixed axial position relative to the injection end (52).

2. The injector as claimed in claim 1, in which the distance between the body stop (130) and the injection opening (32) is adjustable.

3. The injector as claimed in claim 1 or claim 2, in which the distance between the injection end (32) and the reference shoulder (350) is less than 30mm.

4. The injector as claimed in any preceding claim, in which the reference shoulder (350) extends to an outer diameter of at least 6 mm.

## Patentansprüche

1. Injektor, der einen Körper (102) und einen Behälter (104) umfasst, wobei der Körper (102) eine Injektionsöffnung aufweist (32) und einen Antriebsmechanismus (38) enthält, der betätigt werden kann, um den Behälter (104) innerhalb des Körpers (102) zu bewegen und das Medikament aus dem Behälter (104) durch die Injektionsöffnung (32) zu spritzen, wobei der Behälter (104) einen Auslass (106) umfasst und eine Injektionsnadel (108) enthält, die abnehmbar damit verbunden ist, wobei die Injektionsnadel (108) eine Nabe (222) und eine Nadel (224) umfasst, wobei sich die Nabe (222) entlang einer ersten Achse zwischen einem Verbindungsende (214) und einem Nadelende (52) erstreckt, wobei das Verbindungsende (214) ein Verbindungselement zum Verbinden mit dem Behälter (104) umfasst und wobei die Nadel (224) an dem Nadelende (52) mit der Nabe (222) verbunden ist wobei sich die Nadel (224) von der Nabe (222) entlang der ersten Achse zu einem Injektionsende (52) erstreckt, wobei die Injektionsnadel (108) einen Kanal umfasst, der sich von dem Verbindungsende (214) der Nabe (222) bis zu dem Injektionsende (52) der Nadel (224) erstreckt, wobei die Nadel (224) außerdem einen Nadelschutz (16) umfasst, wobei der Nadelschutz (16) abnehmbar mit der Nadelnabe (222) verbunden ist und die Nadel (224) im Wesentlichen abdeckt, wobei der Körper (102) außerdem einen Körperanschlag (130) aufweist, wobei der Körperanschlag (130) eine Schulter (134) aufweist, die sich von dem Körper (130) radial nach innen erstreckt, wobei die Schulter (134) des Körperanschlags (130) eine Öffnung definiert, die von dem Nadelschutz (16) aber nicht von einer Bezugsschulter (350) durchquert werden kann, wobei der Körperanschlag (130) konfiguriert ist, um mit der Bezugsschulter (350) in einen Kontakt zu treten, um die Strecke zu begrenzen, um die sich das Injektionsende (52) der Nadel (224) aus der Injektionsöffnung (32) erstrecken kann, **dadurch gekennzeichnet, dass** die Bezugsschulter (350) an einer Hülse (64) bereitgestellt wird, die auf die Nabe (222) angepasst ist und diese im Wesentlichen umgibt, wobei die Hülse (64) eine separate Komponente ist, die zu der Injektionsnadel hinzugefügt wird und die im Wesentlichen nicht in der Lage ist, sich von dem Injektionsende (52) wegzubewegen, sodass die Bezugsschulter (350) sich relativ zu dem Injektionsende (52) in einer festen axialen Position befindet.

2. Injektor nach Anspruch 1, wobei die Strecke zwischen dem Körperanschlag (130) und der Injektionsöffnung (32) einstellbar ist.

3. Injektor nach Anspruch 1 oder Anspruch 2, wobei die Strecke zwischen dem Injektionsende (32) und der Bezugsschulter (350) geringer als 30 mm ist.

4. Injektor nach einem der vorhergehenden Ansprüche, wobei sich die Bezugsschulter (350) bis zu einem Außendurchmesser von mindestens 6 mm erweitert.

## Revendications

1. Injecteur comprenant un corps (102) et un contenant (104), le corps (102) ayant une ouverture d'injection (32) et comportant un mécanisme d'entraînement (38) qui peut être actionné pour déplacer le contenant (104) à l'intérieur du corps (102) et pour injecter un médicament depuis le contenant (104) à travers l'ouverture d'injection (32), le contenant (104) comprenant une sortie (106) et comportant une aiguille d'injection (108) accouplée à celui-ci de manière amovible, l'aiguille d'injection (108) comprenant un moyeu (222) et une aiguille (224), le moyeu (222) s'étendant le long d'un premier axe entre une extrémité de connexion (214) et une extrémité d'aiguille (52), l'extrémité de connexion (214) comprenant un moyen de connexion pour la connexion au contenant (104) et l'aiguille (224) étant connectée au moyeu (222) au niveau de l'extrémité d'aiguille (52), l'aiguille (224) s'étendant depuis le moyeu (222) le long du premier axe jusqu'à une extrémité d'injection (52), l'aiguille d'injection (108) comprenant un canal s'étendant depuis l'extrémité de connexion (214) du moyeu (222) jusqu'à l'extrémité d'injection (52) de l'aiguille (224), l'aiguille (224) comprenant en outre un protège-aiguille (16), le protège-aiguille (16) étant accouplé de manière amovible au moyeu d'aiguille (222) et couvrant sensiblement l'aiguille (224), le corps (102) comportant en outre une butée de corps (130), la butée de corps (130) comprenant un épaulement (134) s'étendant radialement vers l'intérieur depuis le corps (130),
l'épaulement (134) de la butée de corps (130) définissant une ouverture à travers laquelle peut passer le protège-aiguille (16), mais pas un épaulement de référence (350), la butée de corps (130) étant configurée pour venir en contact avec l'épaulement de référence (350) de manière à limiter la distance suivant laquelle l'extrémité d'injection (52) de l'aiguille (224) s'étend hors de l'ouverture d'injection (32),
**caractérisé en ce que** l'épaulement de référence (350) est prévu sur un manchon (64) qui est ajusté au moyeu (222) et entoure sensiblement ce dernier, le manchon (64) étant un composant séparé ajouté à l'aiguille d'injection et substantiellement incapable de se déplacer à l'écart de l'extrémité d'injection (52) de telle sorte que l'épaulement de référence (350) est dans une position axiale fixe par rapport à l'extrémité d'injection (52).

2. Injecteur selon la revendication 1, dans lequel la distance entre la butée de corps (130) et l'ouverture d'injection (32) est ajustable.

3. Injecteur selon la revendication 1 ou la revendication 2, dans lequel la distance entre l'extrémité d'injection (32) et l'épaulement de référence (350) est inférieure à 30 mm.

4. Injecteur selon l'une quelconque des revendications précédentes, dans lequel l'épaulement de référence (350) s'étend jusqu'à un diamètre extérieur d'au moins 6 mm.
